(19) Europäisches Patentamt / European Patent Office / Office européen des brevets

(11) **EP 3 069 655 B1**

(12) **EUROPEAN PATENT SPECIFICATION**

(45) Date of publication and mention
of the grant of the patent:
**28.08.2024  Bulletin 2024/35**

(21) Application number: **16158919.7**

(22) Date of filing: **07.03.2016**

(51) International Patent Classification (IPC):
***A61B 5/021*** *(2006.01)*  ***A61B 5/022*** *(2006.01)*
***A61B 5/00*** *(2006.01)*

(52) Cooperative Patent Classification (CPC):
**A61B 5/02116; A61B 5/02225; A61B 5/7235**

(54) **MEASURING APPARATUS**

MESSVORRICHTUNG

APPAREIL DE MESURE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.03.2015  JP 2015046701**

(43) Date of publication of application:
**21.09.2016  Bulletin 2016/38**

(73) Proprietor: **Nihon Kohden Corporation
Shinjuku-ku
Tokyo (JP)**

(72) Inventor: **Ukawa, Teiji
Tokyo (JP)**

(74) Representative: **Hoffmann Eitle
Patent- und Rechtsanwälte PartmbB
Arabellastraße 30
81925 München (DE)**

(56) References cited:
**EP-A2- 1 302 155      US-A1- 2005 119 578
US-A1- 2010 298 726**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 069 655 B1

**Description**

BACKGROUND

The presently disclosed subject matter relates to a measuring apparatus, and more particularly to a measuring apparatus which measures the blood pressure of a subject.

In a method of measuring the blood pressure of a subject, an oscillometric blood pressure monitor is widely used. When an oscillometric blood pressure monitor is to be used, the subject attaches a cuff to a predetermined position (preferably, the upper arm). In an oscillometric blood pressure monitor, the cuff pressure is changed between a pressure which is equal to or higher than the systolic blood pressure, and that which is equal to or lower than the diastolic blood pressure, and the systolic blood pressure, the mean blood pressure, and the diastolic blood pressure are calculated from the amplitude change of the pulse wave which is superimposed on the cuff pressure during the change.

[Patent Literature 1] JP-A-2014-230671

[Patent Literature 2] JP-A-10-276994

[Non-patent Patent Literature 1] SHIMAZU Hideaki, Kenko o Sasaeru Iryokiki Kantan de Oku no Fukai Ketsuatsu Sokutei, Clin Eng Vol. 9 No. 1, P26-33

[Non-patent Patent Literature 2] YOSHIKAWA Kimihiko, Heisokusei Domyaku Kokasho Shindan ni Okeru ABI Sokutei to Myakuhakei no Juyosei, DAI 8 KAI RINSHO KETSUATSU MYAKUHA KENKYUKAI SHOREI KENTOKAI 3, P58-P60

[Non-patent Patent Literature 3] Colin Information Service, [online], [Searched on February 23, 2015], Internet <URL: http://www.colin.omron.co.jp/colininfo/pdf/11.pdf>

[Non-patent Patent Literature 4] Denis Chemla et al., "aortic pressure is the geometric mean of systolic and diastolic aortic pressure in resting humans" aortAppl Phusiol 99:2278-2284, 2005

In an oscillometric blood pressure monitor, as described above, a pressure which is equal to or higher than the systolic blood pressure must be applied to the subj ect. Therefore, there are problems in that a large burden is imposed on the patient, and that the measurement time period is long. In the case where the measurement is performed on a subject with advanced arteriosclerosis, an oscillometric blood pressure monitor cannot clearly determine the maximum amplitude of the pulse wave which is superimposed on the cuff pressure. This causes another problem in that, in an oscillometric blood pressure monitor, the accuracy of calculation of the mean blood pressure is low. For example, the blood pressure estimating apparatus disclosed in Patent Literature 1 teaches a technique for reducing a burden on a subject. However, the technique requires a microwave sensor and the like. In the configuration disclosed in Patent Literature 1, therefore, the above-discussed problems cannot be sufficiently solved by an economical configuration.

Patent Literature 2 discloses a measuring apparatus having a configuration in which, in order to enhance the measurement speed, a provisional blood pressure value is displayed. In the technique, the history of the systolic and mean blood pressures is used, and the mean blood pressure is estimated at the timing when the current systolic blood pressure is calculated (Columns 0033 and 0034 of Patent Literature 2) . In the technique, however, the diastolic blood pressure is not considered, and therefore there is a possibility that the blood pressure cannot be correctly estimated.

US 2005/119578 A1 describes electronic blood pressure monitors and methods employing the same to calculate blood pressure. Oscillometry or another method is employed to initially determine a mean blood pressure and a diastolic blood pressure. There is a similarity between an intra-arterial pressure waveform exactly representing blood pressure and a pulse wave form generated when a cuff occludes a site to be measured. This similarity is utilized to obtain blood pressure. More specifically, the cuff occludes a site to be measured, while a pulse wave is detected. From the detected pulse wave' s maximum amplitude an estimated mean arterial pressure is obtained. The obtained estimated mean arterial pressure and the pulse wave's minimum value are regarded as a mean blood pressure and a diastolic blood pressure, respectively. From the pulse wave's maximum value a systolic blood pressure is obtained by an arithmetic operation.

EP 1 302 155 A2 relates to a blood-pressure measuring apparatus including a cuff worn on a body portion of a subject to apply a pressure to the body portion; a changing device which changes the cuff pressure between a pressure lower than a systolic blood pressure of the body portion and higher than a mean blood pressure of the body portion, and a pressure lower than a diastolic blood pressure of the body portion; a determining device which determines a diastolic and a mean blood pressure of the subject based on a heartbeat-synchronous signal detected from the subject while the changing device changes the cuff pressure; a detecting device which detects a cuff pulse wave as an oscillation of the cuff pressure; a pulse-wave-magnitude determining means for operating the changing device to maintain the cuff pressure at a pressure lower than the mean blood pressure of the body portion, and determining a minimal, a mean, and a maximal magnitude of the cuff pulse wave detected in a state in which the cuff pressure is maintained at that pressure; and a systolic-blood-pressure determining means for determining a systolic blood pressure of the subject, based on the determined diastolic and mean blood pressure and the determined minimal, mean, and maximal magnitudes of the cuff pulse wave, according to a fact that the minimal, mean, and maximal magnitudes of the cuff pulse wave correspond to the diastolic, mean, and systolic blood pressure of the subject, respectively.

US 2010/298726 A1 describes a blood pressure monitoring apparatus including a pressing unit which presses a measurement body part of a subject, a sensing unit which senses a sphygmus wave at the measurement body part while the measurement body part is being pressed, a control unit controlling a point of time of stopping the pressing based on an amplitude of the sensed sphygmus wave, and an estimation unit which estimates a blood pressure of the subj ect based on the sphygmus wave sensed before the pressing performed by the pressing unit is stopped.

SUMMARY

The presently disclosed subject matter may provide a measuring apparatus which can solve the various problems caused in the case where an oscillometric blood pressure monitor is used.

The present invention is defined by the independent claims. Preferred embodiments are defined by the dependent claims. Further aspects are provided for facilitating the understanding of the invention.

BRIEF DESCRIPTION OF THE DRAWINGS

Fig. 1 is a block diagram showing the configuration of a measuring apparatus 1 of Embodiment 1.
Fig. 2 is a conceptual view showing the concept of calculation of the diastolic and mean blood pressures by increasing a cuff pressure.
Fig. 3 is a view schematically showing relationships between the pressure and volume of an arterial blood vessel.
Fig. 4 is a conceptual view showing the waveform of a pulse wave.
Fig. 5 is a conceptual view showing the blood pressure waveform.
Figs. 6(A) and 6(B) are conceptual views showing the waveform of a pulse wave.
Fig. 7 is a block diagram showing the configuration of a measuring apparatus 1 of Embodiment 2.
Fig. 8 is a block diagram showing the configuration of a measuring apparatus 1 of Embodiment 4.
Fig. 9 is a view showing the concept of the %MAP.
Fig. 10 is view showing relationships between a cuff pressure and the %MAP.
Fig. 11 is a conceptual view showing pulse waves of cuff pressures which were measured from a subject with advanced arteriosclerosis.

DETAILED DESCRIPTION OF EXEMPLARY EMBODIMENTS

<Embodiment 1>

Hereinafter, an embodiment of the presently disclosed subject matter which will be described with reference to the drawings. The embodiment will be described is aimed to reduce the burden on the subject, and to shorten the measurement time period, and has a configuration which estimates the systolic blood pressure.

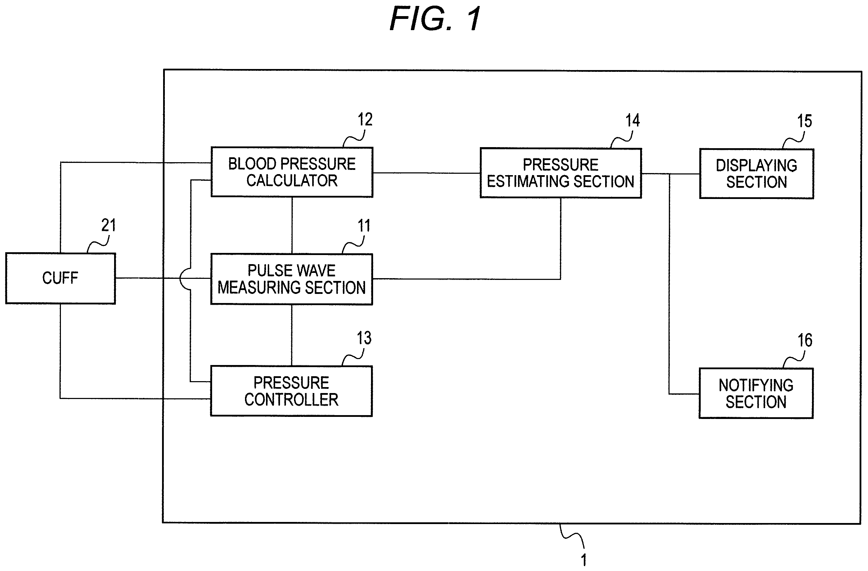

Fig. 1 is a block diagram showing the configuration of a measuring apparatus 1 of the embodiment. The measuring apparatus 1 includes a pulse wave measuring section 11, a blood pressure calculator 12, a pressure controller 13, a blood pressure estimating section 14, a displaying section 15, and a notifying section 16. Although not illustrated, the measuring apparatus 1 adequately includes a storage device (for example, a hard disk drive) and the like. For example, the measuring apparatus 1 may be a patient monitor, a medical telemeter, a blood pressure monitor, or the like. The measuring apparatus 1 is connected to a cuff 21.

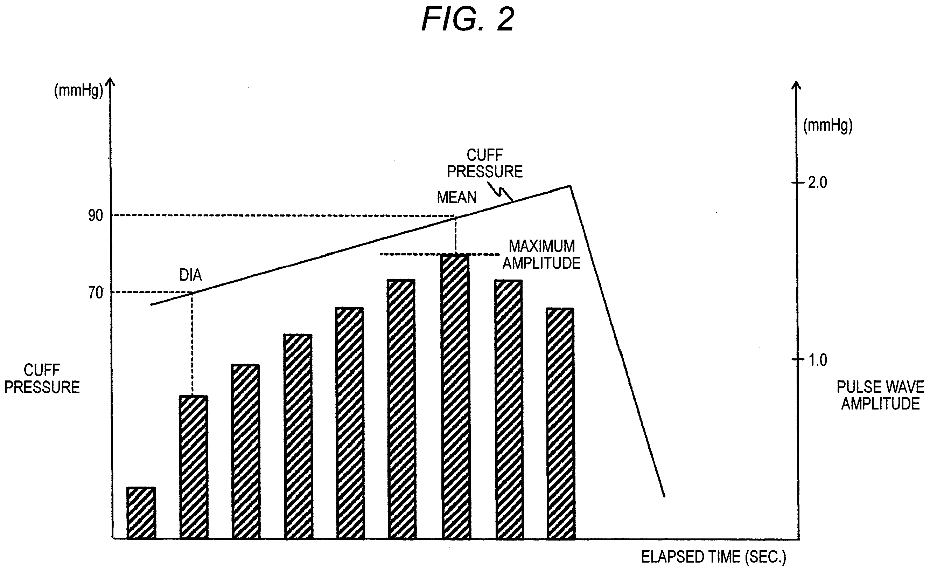

The cuff 21 is an air bag which is used in a related-art non-invasive blood pressure measurement. The cuff 21 is attached to a predetermined portion (preferably, the upper arm) of the subject. The pressure controller 13 performs a control of pressurizing and depressurizing the cuff 21. The pressure controller 13 gradually increases the cuff pressure (the pressure which is applied by the cuff 21 to a predetermined position of the subject) from a state where the cuff pressure is 0, and ends the pressurization (starts the depressurization) after the blood pressure calculator 12 calculates the diastolic and mean blood pressures. The pressure control performed by the pressure controller 13 will be described later in detail with reference to Fig. 2.

The pulse wave measuring section 11 non-invasively measures the pulse wave of the subject. Specifically, the pulse wave measuring section 11 detects the cuff pressure. The pulse wave of the subject is superimposed on the cuff pressure. The pulse wave measuring section 11 performs a signal process on the detected cuff pressure to acquire the pulse wave superimposed on the cuff pressure. Here, the pulse wave measuring section 11 measures the pulse wave during a period when the pressure applied to the cuff 21 has a value which is within a range where the pulse wave component of the vein is not contained. For example, it is considered that a pressure which does not contain the pulse wave component of the vein (a pressure which is higher than the venous pressure) is a pressure that is equal to or higher than 20 mmHg. According to the configuration, influence of the venous pressure can be canceled from the cuff pressure pulse wave, only the pulse wave component related to the arterial pressure can be extracted, and the pulse wave can be accurately measured. Preferably, the pulse wave measuring section 11 measures the pulse wave during pressurization (from 40 mmHg to 50 mmHg) under a pressure which is supposed to be equal to or lower than the diastolic blood pressure. According to the configuration, the artery can be prevented from being excessively closed, and the pulse wave can be accurately measured. The pulse wave measuring section 11 supplies the measured pulse wave to the blood pressure calculator 12 and the blood pressure estimating section 14.

The blood pressure calculator 12 calculates the diastolic and mean blood pressures based on the pulse pressure and the cuff pressure. The method of calculating the diastolic and mean blood pressures will be described with reference to Fig. 2. Fig. 2 is a conceptual view showing the concept of calculation of the diastolic and mean blood pressures by performing pressurization by using the cuff 21.

The blood pressure calculator 12 detects the amplitude (the portion of the bar graph in Fig. 2) of the pulse wave which is superimposed on the cuff pressure in the pressurization process using the cuff 21. The blood pressure calculator 12 detects the cuff pressure corresponding to the maximum amplitude of the pulse wave, as the mean blood pressure (MEAN) . Moreover, the blood pressure calculator 12 calculates the diastolic blood pressure (DIA) based on the maximum amplitude. Although there are various methods of calculating the blood pressure, the blood pressure calculator 12 calculates the diastolic blood pressure (DIA) based on the cuff pressure at a point where the amplitude of the pulse wave is sharply increased. After the diastolic and mean blood pressures are successfully calculated (or after a state where the blood pressures can be calculated is attained), the pressure controller 13 starts depressurization of the cuff 21. That is, the diastolic and mean blood pressures are calculated from the pulse wave which is measured during a period of pressurization at a pressure which is equal to or lower than a pressure that is estimated to be equal to or lower than the systolic blood pressure. Since the pressurization is performed at a pressure which is equal to or lower than the systolic blood pressure, the burden on the subject can be reduced. The blood pressure calculator 12 supplies the calculated diastolic and mean blood pressures to the blood pressure estimating section 14.

Referring again to Fig. 1, the pulse wave, the diastolic blood pressure, and the mean blood pressure are supplied to the blood pressure estimating section 14. The blood pressure estimating section 14 calculates the systolic blood pressure (third blood pressure value) by using the pulse wave, the diastolic blood pressure (first blood pressure value), and the mean blood pressure (second blood pressure value). The blood pressure calculator 12 calculates the systolic blood pressure by using relationships between the pulse wave, the volume of an arterial blood vessel (hereinafter, often referred to as the arterial blood vessel volume), and the blood pressure. Therefore, the relationships between the pulse wave, the arterial blood vessel volume, and the blood pressure will be first described.

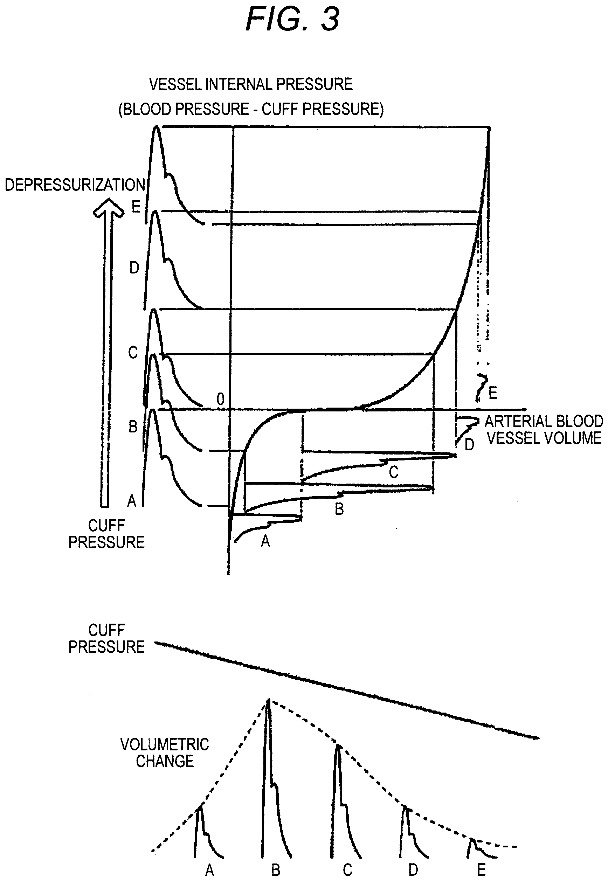

Fig. 3 (cited from Non-patent Literature 1) is a view schematically showing relationships between the pressure and volume of an arterial blood vessel. The ordinate of the figure indicates the blood vessel internal pressure, and the abscissa indicates the arterial blood vessel volume. In the case where the pressure applied to the cuff 21 is higher than the diastolic blood pressure, a blood vessel in which the internal pressure is equal to or lower than the applied pressure is closed.

Therefore, a change of the arterial blood vessel volume shows a discontinuous waveform. Therefore, the relationship between (the value which is obtained by subtracting the cuff pressure from the blood pressure) and (the arterial blood vessel volume) is a nonlinear relationship as shown in Fig. 3.

In the case where the applied pressure of the cuff 21 is lower than the diastolic blood pressure, however, a change of the arterial blood vessel volume shows a shape which is similar to the blood pressure waveform. In the range where the applied pressure of the cuff 21 is lower than the diastolic blood pressure (D or E of Fig. 3), therefore, a change of the arterial blood vessel volume and a blood pressure change have an approximately linear relationship. The relationships between the arterial blood vessel volume and a change of the blood pressure have been described above.

Next, relationships between the pulse wave and the arterial blood vessel volume will be described. A variation of the pulse wave is caused by a change of the cuff volume due to a change of the arterial blood vessel volume. When the cuff pressure is indicated by P, the cuff volume is indicated by V, a change of the cuff volume due to a change of the arterial blood vessel volume is indicated by $\Delta V$, and the amplitude of the pulse wave is indicated by $\Delta P$, the following Exp. (1) holds in accordance with Boyle-Charles's Law.

$$P \times V = (P + \Delta P) \times (V - \Delta V)$$

$$= P \times V + \Delta P \times V - P \times \Delta V - \Delta P \times \Delta V \qquad \text{Exp. (1)}$$

The values of ΔP and ΔV are smaller than those of P and V. Even when the term of ΔP $\times$ ΔV in Exp. (1) above is neglected, therefore, little influence is exerted. Consequently, the following Exp. (2) and Exp. (3) approximately hold.

$$\Delta P \times V = \Delta V \times P \qquad \text{Exp. (2)}$$

$$\Delta P = \Delta V \times P/V \qquad \text{Exp. (3)}$$

When it is assumed that P and V are constant in Exp. (2) and Exp. (3), ΔP is proportional to ΔV. That is, it is possible to consider that the amplitude ΔP of the pulse wave has a similarity relationship to a change of the arterial blood vessel volume.

Summarizing the above description, the followings are obtained:

(Characteristic 1) a change of the arterial blood vessel volume is in approximately linear relationship with that of the blood pressure (in the range where the cuff pressure is equal to or lower than the diastolic blood pressure); and
(Characteristic 2) the amplitude of the pulse wave has a similarity relationship to a change of the arterial blood vessel volume.

When the above two characteristics are considered, the amplitude of the pulse wave is relevant to a change of the blood pressure. Therefore, the blood pressure estimating section 14 focuses attention on the relationships between the amplitude of the pulse wave and the blood pressure, and estimates the systolic blood pressure by using the above-described inputs (the diastolic blood pressure, the mean blood pressure, and the pulse wave). Hereinafter, this will be described in detail.

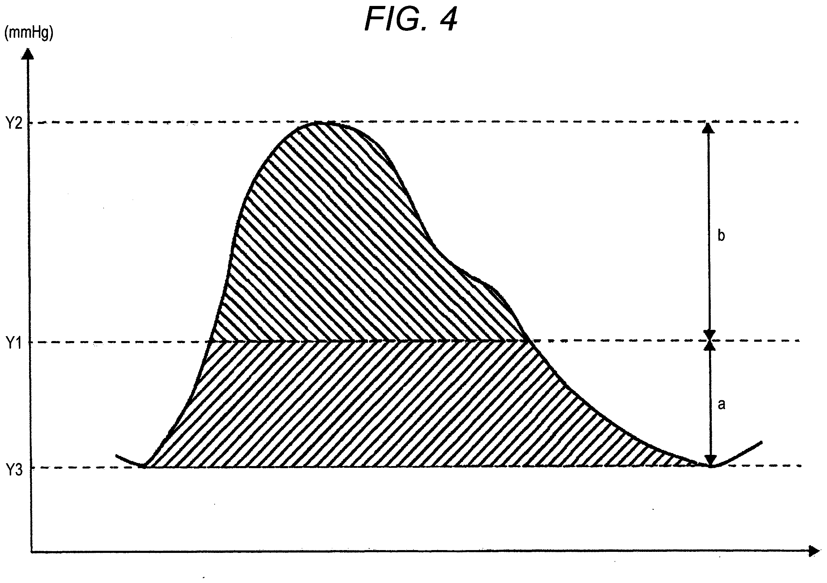

As described above, the pulse wave is relevant to a change of the blood pressure (i.e., the blood pressure waveform). Namely, it is possible to assume that the waveform of the pulse wave is similar to that of the blood pressure. Therefore, the blood pressure estimating section 14 performs the process while assuming that the waveform of the pulse wave is identical with (or similar to) that of the blood pressure. Fig. 4 is a conceptual view showing the waveform of the pulse wave.

In one amplitude of the waveform of the pulse wave shown in Fig. 4, the blood pressure estimating section 14 calculates a pressure value Y1 (mmHg) by which the area of the one amplitude waveform is divided so that the two divided waveform sections have the same area. Then, the blood pressure estimating section 14 calculates the ratio of a : b in the figure (Y1 - minimum pressure value Y3 in the one amplitude : maximum pressure value Y2 in the one amplitude - Y1). The following description is made assuming that the ratio of a : b is 1 : 2.

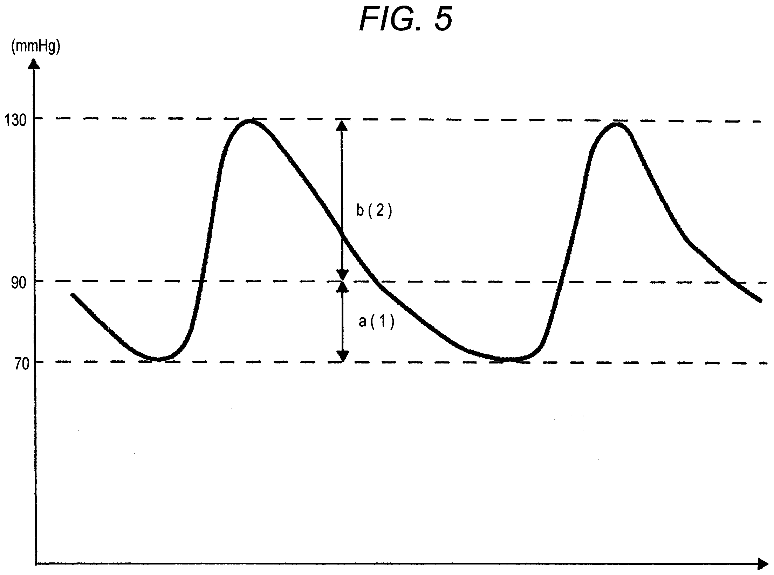

Next, the blood pressure estimating section 14 estimates the systolic blood pressure by using the ratio, the diastolic blood pressure, and the mean blood pressure. Fig. 5 is a conceptual view showing the blood pressure waveform. As described above, it is possible to assume that the waveform of the blood pressure is similar to that of the pulse wave (Fig. 4). The blood pressure calculator 12 calculates the diastolic blood pressure and the mean blood pressure as described above. In the following description, it is assumed that the diastolic blood pressure is 70 mmHg and the mean blood pressure is 90 mmHg. Here, the mean blood pressure is a value by which, in the case where one amplitude of the waveform of the blood pressure is considered as an area, the area is divided into halves. The ratio (a : b) which is calculated in Fig. 4 is 1 : 2. As described above, it is possible to assume that the waveform of the blood pressure is similar to that of the pulse wave (Fig. 4). Therefore, the blood pressure estimating section 14 assumes that (diastolic blood pressure - mean blood pressure) : (systolic blood pressure - mean blood pressure) = 1 : 2. Based on the ratio (a : b = 1 : 2), the diastolic blood pressure (70 mmHg), and the mean blood pressure (90 mmHg), the blood pressure estimating section 14 estimates that the systolic blood pressure is 130 mmHg (90 mmHg + (90 mmHg - 70 mmHg) $\times$ (2/1)).

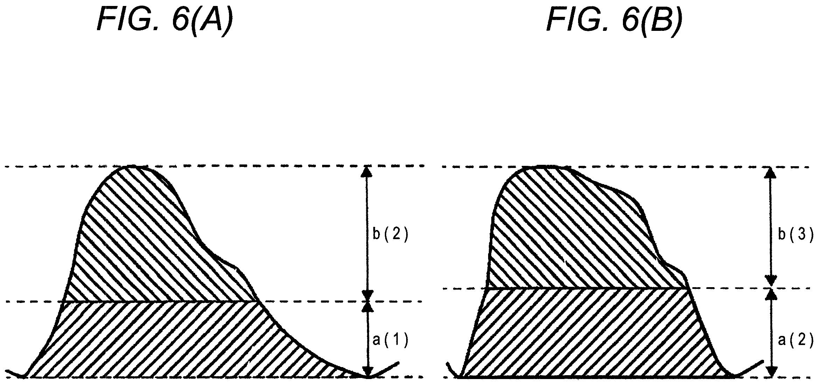

The method of estimating the systolic blood pressure in the blood pressure estimating section 14 will be described in more detail. The above-described blood pressure estimating section 14 estimates the systolic blood pressure based on the shape of the waveform of the pulse wave. Various examples of the shape of the pulse wave, and the method of estimating the systolic blood pressure based thereon will be described with reference to Figs. 6(A) and 6(B).

Fig. 6(A) shows a first example of the waveform of the pulse wave. In Fig. 6(A), the ratio of a : b which divides the area of the waveform into halves is 1 : 2. Fig. 6(B) shows a second example of the waveform of the pulse wave. In Fig. 6(B), the ratio of a : b which divides the area of the waveform into halves is 2 : 3. In cases having different waveform shapes of the pulse wave, namely, the ratio of a : b which divides the area of the waveform into halves is varied. As described above, it is possible to assume that the waveform of the blood pressure is similar to that of the pulse wave (Fig. 4). Therefore, the blood pressure estimating section 14 performs estimation while assuming that, depending on the waveform of the pulse wave, also relationships of blood pressures (the diastolic blood pressure, the mean blood pressure, and the systolic blood pressure) are changed. In other words, the blood pressure estimating section 14 estimates the systolic blood pressure while treating the shape of the pulse wave as a ratio indicating the relationships of the blood pressure values (the diastolic blood pressure, the mean blood pressure, and the systolic blood pressure).

Referring again to Fig. 1, the displaying section 15 displays the diastolic and mean blood pressures which are calculated by the blood pressure calculator 12, and the systolic blood pressure which is estimated by the blood pressure estimating section 14, on a display device or the like disposed in the measuring apparatus 1. In the case where the blood pressure value (the diastolic blood pressure, the mean blood pressure, or the systolic blood pressure) is abnormal, the notifying section 16 outputs an alarm sound.

Then, effects of the measuring apparatus 1 of the embodiment will be described. As described above, the measuring apparatus 1 has the configuration in which the systolic blood pressure (third blood pressure value) is calculated based on two blood pressure values (the diastolic blood pressure (first blood pressure value) and the mean blood pressure (second blood pressure value)). During the calculation of the diastolic and mean blood pressures, the pressure controller 13 pressurizes the cuff 21. In the pressurization process, the blood pressure calculator 12 calculates the diastolic and mean blood pressures. After the two blood pressure values are calculated, the pressurization is ended (Fig. 2). In other words, the pressurization is not performed at a pressure which is estimated to be equal to or higher than the systolic blood pressure. According to the configuration, as compared with a blood pressure measurement by a related-art oscillometric method, the measurement time period is shortened, and the burden on the subject (i.e., the cuff pressure applied to the predetermined portion) can be reduced.

<Embodiment 2>

The measuring apparatus 1 of the embodiment is characterized in that the systolic blood pressure is estimated by using blood pressure values which are measured in the past. With respect to the embodiment, points which are different from Embodiment 1 will be described.

First, an example of a use case where the measuring apparatus 1 of the embodiment is required will be described. In the case where anesthesia is applied in an operating room, the anesthetist frequently measures the blood pressure of the subject in order to determine the onset of anesthesia. In this case, the anesthetist often emphasizes the recognition of transition of the blood pressure from the previous measurement (i.e., whether the blood pressure is in a rising trend or a falling trend), than a correct measurement value. The measuring apparatus 1 which is effective in the case where the previous result of the blood pressure measurement remains as described above, and transition of the blood pressure is to be immediately known will be described.

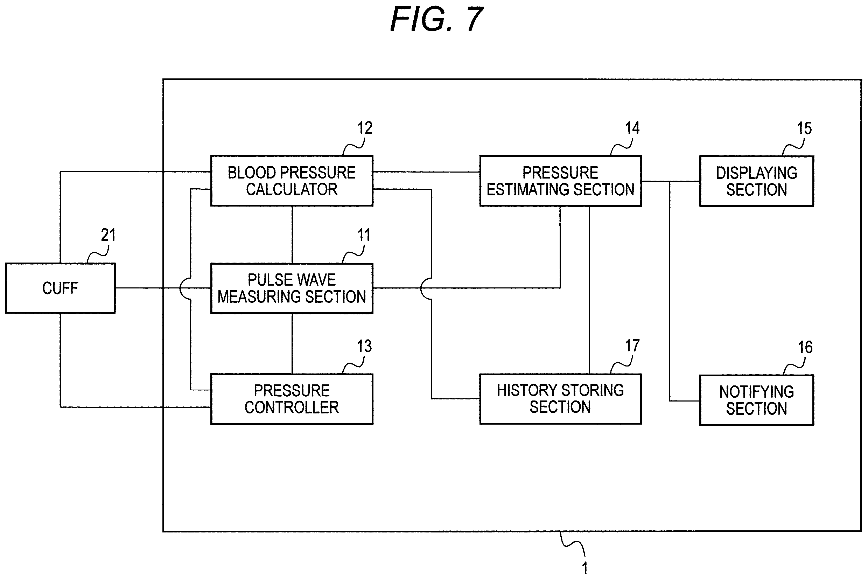

Fig. 7 is a block diagram showing the configuration of a measuring apparatus 1 of the embodiment. The measuring apparatus 1 shown in Fig. 7 is configured so as to further have a history storing section 17 in addition to the configuration of Fig. 1. The processes of the processing sections are different from those of Embodiment 1 in the following points.

The blood pressure calculator 12 and the pressure controller 13 perform a related-art blood pressure measurement using the oscillometric method, at least one time on the subject. The blood pressure calculator 12 and the pressure controller 13 store blood pressure values (the diastolic blood pressure, the mean blood pressure, and the systolic blood pressure) which are calculated by using the oscillometric method, in the history storing section 17. That is, the pressure controller 13 pressurizes the cuff 21 to a pressure which is estimated to be equal to or higher than the systolic blood pressure, and thereafter depressurizes the cuff 21. In the depressurization process, the blood pressure calculator 12 calculates the diastolic, mean, and systolic blood pressures based on the amplitude of the pulse wave. The blood pressure calculator 12 stores the calculated blood pressure values (the diastolic blood pressure, the mean blood pressure, and the systolic blood pressure) as reference values in the history storing section 17.

Hereinafter, a blood pressure value stored in the history storing section 17 is generally referred to as a reference blood pressure value. The diastolic blood pressure, mean blood pressure, and systolic blood pressure which are stored in the history storing section 17 are referred to as the reference diastolic blood pressure, the reference mean blood pressure, and the reference systolic blood pressure, respectively. The history storing section 17 may be realized by, for example, a hard disk drive which is disposed in the measuring apparatus 1.

Thereafter, the measuring apparatus 1 calculates the blood pressure of the subject periodically or in accordance with manual instructions. In this case, the measuring apparatus 1 estimates the systolic blood pressure without performing pressurization at a pressure which is equal to or higher than the systolic blood pressure, similarly with Embodiment 1.

This will be described in detail. The pressure controller 13 gradually increases the cuff pressure (the pressure which is applied by the cuff 21 to the predetermined portion of the subject) from a state where the cuff pressure is 0, and ends the pressurization (starts the depressurization) after the blood pressure calculator 12 calculates the diastolic and mean blood pressures. The blood pressure estimating section 14 estimates the systolic blood pressure based on the calculated diastolic and mean blood pressures, and the reference blood pressure value values (the reference diastolic blood pressure, the reference mean blood pressure, and the reference systolic blood pressure) read out from the history storing section 17.

The estimation is performed by the below-described method. In the following description, it is assumed that the reference blood pressure values have the following values, respectively:

- reference diastolic blood pressure = 70 mmHg
- reference mean blood pressure = 90 mmHg
- reference systolic blood pressure = 130 mmHg.

From the above reference blood pressure value values, the blood pressure estimating section 14 calculates a ratio (reference mean blood pressure - reference diastolic blood pressure : reference systolic blood pressure - reference mean blood pressure) as 1 : 2. The blood pressure estimating section 14 estimates the systolic blood pressure so as to attain coincidence with the ratio. For example, it is assumed that the mean blood pressure calculated by the blood pressure calculator 12 is 92 mmHg, and the diastolic blood pressure is 70 mmHg. In this case, the blood pressure estimating section 14 estimates that the current systolic blood pressure is 136 mmHg (92 mmHg + (92 mmHg - 70 mmHg) $\times$ (2/1)).

The processes other than the above are similar to those of Embodiment 1. Then, effects of the measuring apparatus 1 of the embodiment will be described. The measuring apparatus 1 estimates the systolic blood pressure by using the reference blood pressure values, and the diastolic and mean blood pressures which are calculated by the blood pressure calculator 12. The blood pressure calculator 12 previously calculates the reference blood pressure values (the reference diastolic blood pressure, the reference mean blood pressure, and the reference systolic blood pressure) by using a related-art oscillometric method. The calculation is performed by using the oscillometric method, and therefore the reference blood pressure values are correct. The blood pressure estimating section 14 estimates the systolic blood pressure by using the correct reference blood pressure values. Therefore, the systolic blood pressure can be accurately estimated.

In the above-described blood pressure measurement in introduction of anesthesia, particularly, it is requested to rapidly perform the blood pressure measurement. Even in such a case, the measuring apparatus 1 can promptly calculate the systolic blood pressure corresponding to the blood pressure trend of the subject.

<Embodiment 3>

The measuring apparatus 1 of the embodiment estimates the systolic blood pressure by using past blood pressure values (reference blood pressure values) similarly with Embodiment 2. With respect to the measuring apparatus 1 of the embodiment, points which are different from Embodiment 2 will be described.

The configuration of the measuring apparatus 1 is similar to that of Embodiment 2 (Fig. 7). The blood pressure calculator 12 and the pressure controller 13 perform a related-art blood pressure measurement using the oscillometric method, at least one time on the subject. The calculated reference blood pressure values are stored in the history storing section 17. At the timing (a substantially same timing) of the blood pressure measurement performed by the oscillometric method, the pulse wave measuring section 11 measures the pulse wave which is superimposed on the cuff pressure, and stores it in the history storing section 17.

In the following description, it is assumed that the reference blood pressure values have the following values, and the ratio of a : b (Fig. 5) which divides the area of the pulse wave waveform in the measurement by the oscillometric method into halves has the following value:

- reference diastolic blood pressure = 80 mmHg
- reference mean blood pressure = 98 mmHg
- reference systolic blood pressure = 125 mmHg
- ratio of a : b = 1 : 2.

Here, the ratio based on the measured values (reference blood pressure values calculated by using the oscillometric method) is (reference mean blood pressure - reference diastolic blood pressure : reference systolic blood pressure - reference mean blood pressure) = 2 : 3. Therefore, the blood pressure estimating section 14 calculates a correction value for correcting the ratio of a : b calculated from the pulse wave, as 6/5 (= (2/(2 + 3)/(1/(1 + 2))).

The preparatory process using the oscillometric method has been described. Similarly with Embodiments 1 and 2, then, the blood pressure measurement and the pulse wave measurement in the pressurization process are performed at an arbitrary timing. Namely, the blood pressure calculator 12 calculates the diastolic blood pressure and the mean blood pressure in the pressurization process. After the calculation of the mean blood pressure, the pressurization is promptly ended. Moreover, the pulse wave measuring section 11 acquires the pulse wave superimposed on the cuff pressure.

The blood pressure estimating section 14 estimates the systolic blood pressure by using the diastolic and mean blood pressures calculated by the blood pressure calculator 12, the pulse wave measured by the pulse wave measuring section 11, and the correction value (6/5). The diastolic and mean blood pressures calculated by the blood pressure calculator 12 are assumed to have the following values., respectively Here, it is assumed that the shape of the pulse wave is changed, and the ratio of a : b (Fig. 5) which divides the area of the pulse wave waveform into halves has the following value:

- diastolic blood pressure = 80 mmHg
- mean blood pressure = 98 mmHg
- ratio of a : b = 3 : 7.

First, the blood pressure estimating section 14 corrects the above-described ratio (3 : 7) by using the correction value (6/5) in the following manner:

- correction ratio of a' : b' = 3 $\times$ (6/5) : (3 + 7) - 3 $\times$ (6/5) = 3.6 : 6.4.

The blood pressure estimating section 14 estimates the systolic blood pressure by using the correction ratio (3.6 : 6.4), the diastolic blood pressure (80 mmHg), and the mean blood pressure (98 mmHg) in the following manner:

- systolic blood pressure = 98 mmHg + (98 mmHg - 80 mmHg) $\times$ (6.4/3.6) = 130 mmHg.

The other processes may be similar to those of Embodiment 1. Then, effects of the measuring apparatus 1 of the embodiment will be described. As described above, the measuring apparatus 1 obtains the correction value by using the reference blood pressure values which are previously acquired by using the oscillometric method. From the pulse wave and the correction value, then, the measuring apparatus 1 calculates the correction ratio, and estimates the systolic blood pressure by using the correction ratio. The reference blood pressure values are calculated by using the oscillometric method which is used in a related-art blood pressure measurement, and therefore have an accurate value. Even in the case where the shape of the pulse wave is changed by lapse of time, when the correction is performed by using the reference blood pressure values, the blood pressure estimating section 14 can correctly estimate the systolic blood pressure.

<Embodiment 4>

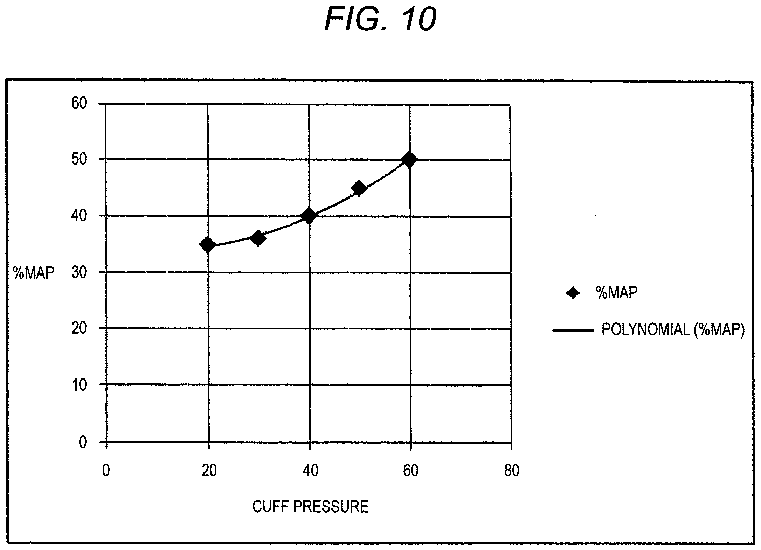

The embodiment is characterized in that the systolic blood pressure is estimated by referring the %MAP (Mean Artery Pressure). With respect to the measuring apparatus 1 of the embodiment, points which are different from the other embodiments will be described.

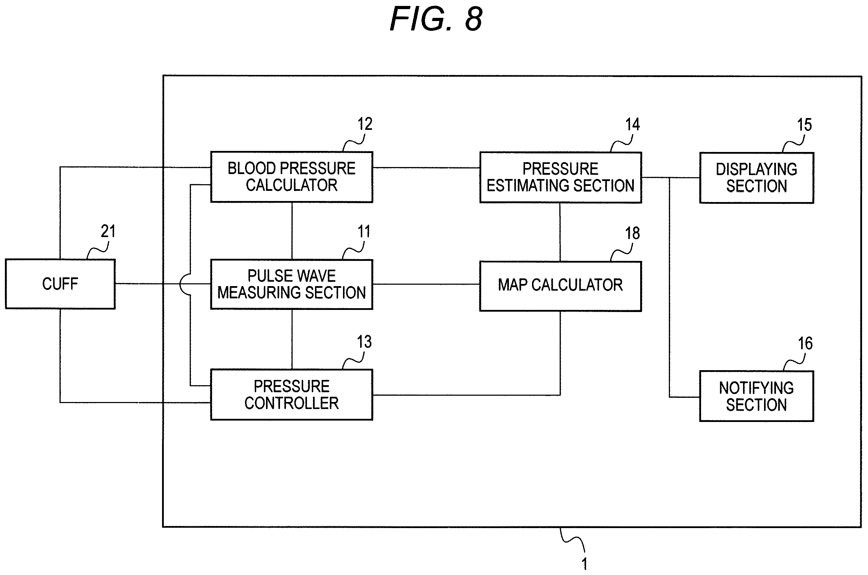

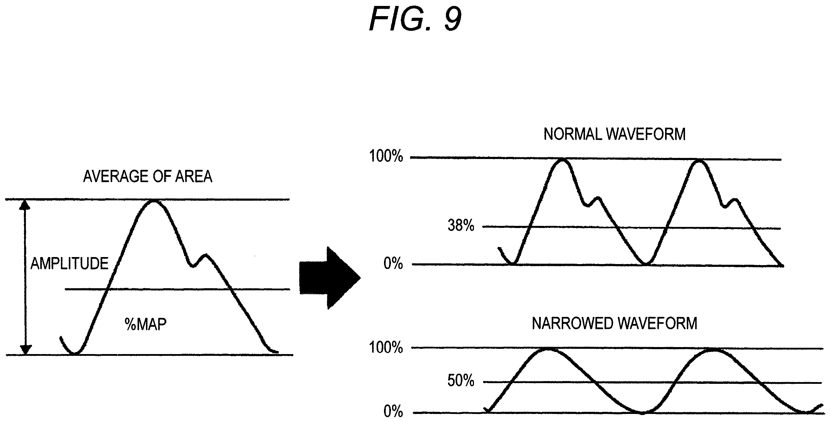

Fig. 8 shows the configuration of the measuring apparatus 1 of the embodiment. As shown in Fig. 8, the measuring apparatus 1 of the embodiment further includes a MAP calculator 18 in addition to the configuration of Fig. 1. In advance to the description of the MAP calculator 18, the %MAP will be described. As shown in Fig. 9, the %MAP is a value which is obtained by dividing the average of the area of the waveform, and expressing the quotient in percentage (Fig. 1 of Non-patent Literature 2). In the case where a blood vessel is narrowed or closed, the value of the %MAP is increased (see Fig. 9) . When the %MAP is smaller than 45%, it is usually determined that the state is normal.
Next, relationships between the cuff pressure (the pressurizing force of the pressure controller 13) and the %MAP will be described with reference to Fig. 10. In Fig. 10, the abscissa indicates the cuff pressure (the pressure which is applied to the predetermined portion of the subject by the cuff 21), and the ordinate indicates the %MAP.
In the case where the cuff pressure is high (for example, 40 mmHg or higher), it is considered that a blood vessel collapses, and therefore the %MAP is large. By contrast, the lower the cuff pressure, the higher the intravascular pressure. That is, a state where little external pressure is applied to a blood vessel is obtained. Therefore, the lower the cuff pressure, the more the relationships between the intravascular pressure and the sectional area of a blood vessel become linear.
Based on the relationships shown in Fig. 10 and between the cuff pressure and the %MAP, the MAP calculator 18 calculates (estimates) the %MAP in the case where the cuff pressure is 0 (the pressurization by the pressure controller 13 is canceled) . An example of the estimation technique will be described.
First, the pulse wave measuring section 11 supplies the pulse wave superimposed on the cuff pressure, to the MAP calculator 18. Based on the pulse wave, the MAP calculator 18 calculates relationships corresponding to Fig. 10 and between the cuff pressure and the %MAP. Then, the MAP calculator 18 calculates an estimated value of the %MAP in the case where the cuff pressure is 0. For example, the MAP calculator 18 calculates an approximate expression of the %MAP such as shown in Fig. 10, and, from the approximate expression, calculates the %MAP in the case where the cuff pressure is 0. The lower the cuff pressure, the more the relationships of the approximate expression become linear. Therefore, the MAP calculator 18 may produce an approximate expression with setting the range where the cuff pressure is equal to or less than a predetermined value, as the object. The MAP calculator 18 supplies the calculated %MAP to the blood pressure estimating section 14.

The blood pressure estimating section 14 estimates the systolic blood pressure by using the diastolic and mean blood pressures calculated by the blood pressure calculator 12, and the %MAP. An example in which the diastolic and mean blood pressures, and the estimated value of the %MAP in the case where the cuff pressure is 0 have the following values, respectively will be described:

- diastolic blood pressure = 70 mmHg
- mean blood pressure = 100 mmHg
- %MAP = 40%.

In the case of the above values, the blood pressure estimating section 14 estimates the systolic blood pressure in the following manner:

- systolic blood pressure = 70 mmHg + (100 mmHg - 70 mmHg)/0.4 = 145 mmHg.

Then, effects of the measuring apparatus 1 of the embodiment will be described. The measuring apparatus 1 of the embodiment estimates the systolic blood pressure by using the relationships between the cuff pressure and the %MAP. As described above, the measuring apparatus 1 calculates the %MAP in the case where the cuff pressure is 0, from the relationships between the cuff pressure and transition of the %MAP. Then, the measuring apparatus 1 estimates the systolic blood pressure by using the %MAP. The %MAP is an index related to the average of the area of the pulse wave. Even in the case where the shape of the pulse wave is changed by lapse of time, when the %MAP is used, therefore, the blood pressure estimating section 14 can accurately estimate the systolic blood pressure.

<Embodiment 5>

Although Embodiments 5 to 8 which will be described are the measuring apparatus 1 which estimates the mean blood pressure, the processes of the embodiments correspond to those of Embodiment 1 to 4, respectively. The measuring apparatus 1 of Embodiment 5 has a configuration in which the mean blood pressure is estimated based on the diastolic blood pressure, the systolic blood pressure, and the pulse wave. The estimation technique basically corresponds to Embodiment 1. Therefore, points which are different from Embodiment 1 will be described.

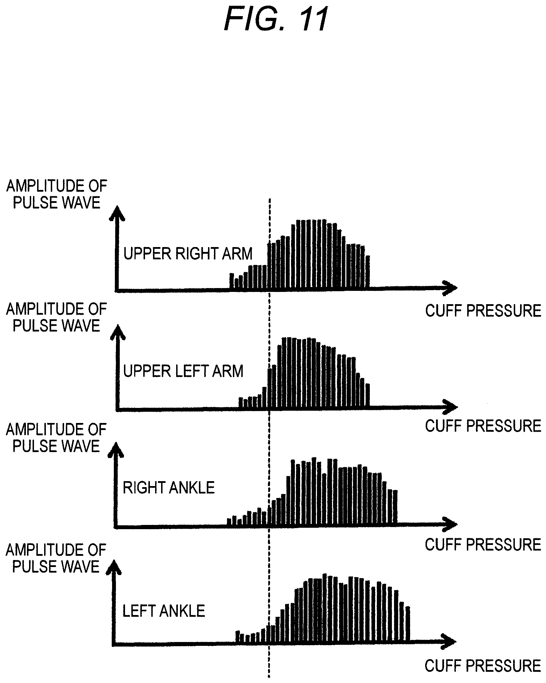

First, an example of a use case where the measuring apparatus 1 of the embodiment is assumed to be used will be described. The subject is assumed to be a patient with advanced arteriosclerosis. With respect to such a subject, a related-art oscillometric blood pressure monitor cannot sometimes clearly determine the maximum amplitude of the pulse wave which is superimposed on the cuff pressure. Fig. 11 (cited from Non-patent Literature 3) is a conceptual view showing pulse waves which were measured from a subject with advanced arteriosclerosis. As illustrated, the point where the amplitude is maximum is not clear. Therefore, a related-art oscillometric blood pressure monitor cannot correctly calculate the mean blood pressure which is a blood pressure corresponding to the point where the amplitude is maximum. It is a main object of the measuring apparatus 1 of the embodiment to correctly calculate the mean blood pressure even from such a subject.

The configuration of the measuring apparatus 1 of the embodiment is similar to that shown in Fig. 1, but the operation is different in the following points. The blood pressure calculator 12 calculates the diastolic and systolic blood pressures. Therefore, the pressure controller 13 applies a pressure which is equal to or higher than the systolic blood pressure, to a predetermined position (preferably, the upper arm) of the subject. The method of calculating the diastolic and systolic blood pressures by the blood pressure calculator 12 may be similar to the calculation method in a related-art oscillometric method.

In the following description, it is assumed that the systolic blood pressure is 130 mmHg, the diastolic blood pressure is 70 mmHg, and the above-described ratio of a : b (similar to Fig. 4, a ratio in one amplitude of the pulse wave) is 1 : 2. From these values, the blood pressure estimating section 14 estimates that the mean blood pressure is 90 mmHg (70 mmHg + (130 mmHg - 70 mmHg) $\times$ (1/3)).

Then, effects of the measuring apparatus 1 of the embodiment will be described. In the blood pressure measurement using a related-art oscillometric method, as described above, the mean blood pressure of a subject with advanced arteriosclerosis cannot be sometimes correctly measured. By contrast, as described above, the measuring apparatus 1 of the embodiment estimates the mean blood pressure by using the systolic and diastolic blood pressures and the ratio which is calculated from the pulse wave. That is, the measuring apparatus 1 estimates the mean blood pressure without using the oscillation amplitude. Even in the case where the maximum value of the oscillation amplitude cannot be clearly identified, therefore, the correct mean blood pressure can be calculated.

<Embodiment 6>

The measuring apparatus 1 of the embodiment has a configuration in which the mean blood pressure is estimated based on the diastolic and systolic blood pressures and reference blood pressure values. The estimation technique basically corresponds to Embodiment 2. Therefore, points which are different from Embodiment 2 will be described.

The configuration of the measuring apparatus 1 is similar to that shown in Fig. 7. An example of a use case where the measuring apparatus 1 of the embodiment is assumed to be used is identical with Embodiment 5 (a subject with advanced arteriosclerosis). Hereinafter, processes which are different from those of Embodiment 2 will be described with reference to Fig. 7.

The blood pressure calculator 12 and the pressure controller 13 perform a related-art blood pressure measurement using the oscillometric method, at least one time on the subject. The blood pressure calculator 12 and the pressure controller 13 store the reference blood pressure values (the reference diastolic blood pressure, the reference mean blood pressure, and the reference systolic blood pressure) which are calculated by using the oscillometric method, in the history storing section 17.

Thereafter, the measuring apparatus 1 calculates the blood pressure of the subject periodically or in accordance with manual instructions. The blood pressure calculator 12 calculates the diastolic and systolic blood pressures based on the pulse pressure and the cuff pressure. The blood pressure estimating section 14 estimates the mean blood pressure based on the calculated diastolic and systolic blood pressures, and the reference blood pressure values (the reference diastolic blood pressure, the reference mean blood pressure, and the reference systolic blood pressure) read out from the history storing section 17.

The estimation is performed by the below-described method. In the following description, it is assumed that the reference blood pressure values are as follows:

- reference diastolic blood pressure = 70 mmHg
- reference mean blood pressure = 90 mmHg
- reference systolic blood pressure = 130 mmHg.

From the above reference blood pressure values, the blood pressure estimating section 14 calculates a ratio (reference mean blood pressure - reference diastolic blood pressure : reference systolic blood pressure - reference mean blood pressure) as 1 : 2. The blood pressure estimating section 14 estimates the mean blood pressure so as to attain coincidence with the ratio. For example, it is assumed that the diastolic blood pressure calculated by the blood pressure calculator 12 is 70 mmHg, and the systolic blood pressure is 133 mmHg. In this case, the blood pressure estimating section 14 estimates that the current mean blood pressure is 91 mmHg (70 mmHg + (133 mmHg - 70 mmHg) × (1/3)).

The other processes are similar to those of Embodiment 2. Then, effects of the measuring apparatus 1 of the embodiment will be described. The measuring apparatus 1 estimates the mean blood pressure by using the reference blood pressure values, the diastolic blood pressure, and the systolic blood pressure. The blood pressure estimating section 14 estimates the mean blood pressure by using the correct reference blood pressure values which are acquired by the oscillometric method. Therefore, the mean blood pressure can be accurately estimated.

<Embodiment 7>

The measuring apparatus 1 of the embodiment estimates the mean blood pressure by using past blood pressure values (reference blood pressure values) similarly with Embodiment 6. The processes of the measuring apparatus 1 of Embodiment 7 correspond to those of the measuring apparatus 1 of Embodiment 3. Therefore, points which are different from Embodiment 3 will be described.

The configuration of the measuring apparatus 1 is similar to that of Embodiment 2 (Fig. 7). The blood pressure calculator 12 and the pressure controller 13 perform a related-art blood pressure measurement using the oscillometric method, at least one time on the subject. The calculated reference blood pressure values are stored in the history storing section 17. At the timing (a substantially same timing) of the blood pressure measurement performed by the oscillometric method, the pulse wave measuring section 11 measures the pulse wave which is superimposed on the cuff pressure, and stores it in the history storing section 17.

In the following description, it is assumed that the reference blood pressure values have the following values, respectively, and the ratio of a : b (Fig. 5) which divides the area of the pulse wave waveform in the measurement by the oscillometric method into halves has the following value:

- reference diastolic blood pressure = 80 mmHg
- reference mean blood pressure = 98 mmHg

- reference systolic blood pressure = 125 mmHg
- ratio of a : b = 1 : 2.

Here, the ratio based on the measured values (reference blood pressure values calculated by using the oscillometric method) is (reference mean blood pressure - reference diastolic blood pressure : reference systolic blood pressure - reference mean blood pressure) = 2 : 3. Therefore, the blood pressure estimating section 14 calculates a correction value for correcting the ratio of a : b calculated from the pulse wave, as 6/5 (= (2/(2 + 3)/(1/(1 + 2)).

The preparatory process using the oscillometric method has been described. Then, the blood pressure calculator 12 measures the blood pressures (the diastolic blood pressure and the systolic blood pressure) at an arbitrary timing. Moreover, the pulse wave measuring section 11 acquires the pulse wave superimposed on the cuff pressure.

The blood pressure estimating section 14 estimates the mean blood pressure by using the diastolic and systolic blood pressures calculated by the blood pressure calculator 12, the pulse wave measured by the pulse wave measuring section 11, and the correction value (6/5). The diastolic and systolic blood pressures calculated by the blood pressure calculator 12 are assumed to have the following values, respectively. Here, it is assumed that the shape of the pulse wave is changed, and the ratio of a : b (Fig. 5) which divides the area of the pulse wave waveform into halves has the following value:

- diastolic blood pressure = 80 mmHg
- systolic blood pressure = 130 mmHg
- ratio of a : b = 3 : 7.

First, the blood pressure estimating section 14 corrects the above-described ratio (3 : 7) by using the correction value (6/5) in the following manner:

- correction ratio of a' : b' = 3 × (6/5) : (3 + 7) - 3 × (6/5) = 3.6 : 6.4.

The blood pressure estimating section 14 estimates the mean blood pressure by using the correction ratio (3.6 : 6.4), the diastolic blood pressure (80 mmHg), and the systolic blood pressure (130 mmHg) in the following manner:

- mean blood pressure = 80 mmHg + (130 mmHg - 80 mmHg) × (3.6/ (6.4 + 3.6) = 98 mmHg.

Then, effects of the measuring apparatus 1 of the embodiment will be described. As described above, the measuring apparatus 1 obtains the correction value by using the reference blood pressure values which are previously acquired by using the oscillometric method. From the pulse wave and the correction value, then, the measuring apparatus 1 calculates the correction ratio, and estimates the mean blood pressure by using the correction ratio. Even in the case where the shape of the pulse wave is changed by lapse of time, when the correction is performed by using the reference blood pressure values, the blood pressure estimating section 14 can correctly estimate the mean blood pressure.

<Embodiment 8>

The embodiment is characterized in that the mean blood pressure is estimated by referring the %MAP (Mean Artery Pressure). The processes of the measuring apparatus 1 of Embodiment 8 correspond to those of Embodiment 4. Therefore, points which are different from Embodiment 4 will be described.

The configuration of the measuring apparatus 1 is similar to that of Embodiment 4 (Fig. 8). Namely, the measuring apparatus 1 has the MAP calculator 18. The MAP calculator 18 calculates the %MAP in a manner similar to Embodiment 4.

The blood pressure calculator 12 calculates the diastolic and systolic blood pressures by using a related-art oscillometric method. The blood pressure estimating section 14 estimates the mean blood pressure by using the diastolic and systolic blood pressures calculated by the blood pressure calculator 12, and the estimated value of the %MAP. An example in which the diastolic and systolic blood pressures, and the estimated value of the %MAP in the case where the cuff pressure is 0 (the pressurization by the pressure controller 13 is canceled) have the following values, respectively will be described:

- diastolic blood pressure = 70 mmHg
- systolic blood pressure = 120 mmHg
- %MAP = 40%.

In the case of the above values, the blood pressure estimating section 14 estimates the mean blood pressure in the following manner:

- mean blood pressure = 70 mmHg + (120 mmHg - 70 mmHg) $\times$ (0.4) = 90 mmHg.

Then, effects of the measuring apparatus 1 of the embodiment will be described. The measuring apparatus 1 of the embodiment estimates the mean blood pressure by using the relationships between the cuff pressure and the %MAP. As described above, the measuring apparatus 1 calculates the %MAP in the case where the cuff pressure is 0, from the relationships between the cuff pressure and transition of the %MAP. Then, the measuring apparatus 1 estimates the mean blood pressure by using the %MAP. The %MAP is an index related to the average of the area of the pulse wave. When the %MAP is used, therefore, the blood pressure estimating section 14 can accurately estimate the mean blood pressure.

(Summary of processes of measuring apparatus 1 of Embodiments 1 to 8)

The summary of processes common to the measuring apparatuses 1 of the above-described Embodiments 1 to 8 will be again described. The blood pressure calculator 12 calculates two blood pressure values (first and second blood pressure values) by using the oscillometric method. The calculation method is not limited to the oscillometric method. The blood pressure calculator 12 may use another technique as far as two blood pressure values are correctly obtained. In Embodiment 1 to 4, the diastolic blood pressure value is calculated as the first blood pressure value, and the mean blood pressure value is calculated as the second blood pressure value . In Embodiment 5 to 8, the diastolic blood pressure value is calculated as the first blood pressure value, and the systolic blood pressure value is calculated as the second blood pressure value.

The blood pressure estimating section 14 estimates the third blood pressure value based on the first and second blood pressure values and the ratio information indicative of the relationships of the difference levels of the blood pressure values. In Embodiment 1 to 4, the systolic blood pressure is estimated as the third blood pressure value, and, in Embodiment 5 to 8, the mean blood pressure is estimated as the third blood pressure value. Here, the ratio information is indicated by the ratio of levels of (the mean blood pressure - the diastolic blood pressure) and (the systolic blood pressure - the mean blood pressure). Of course, the ratio information may be regarded as the ratio of levels of (the systolic blood pressure - the diastolic blood pressure) and (the mean blood pressure - the diastolic blood pressure).

In the embodiments, the ratio information is calculated in the different methods. In Embodiments 1 and 5, the pulse wave measuring section 11 acquires the pulse wave. The blood pressure estimating section 14 focuses attention on that there is relevance between the amplitude of the pulse wave and a change of the blood pressure, and calculates the ratio information (a : b in Fig. 5) based on the shape of the pulse wave. The blood pressure estimating section 14 estimates the third blood pressure value by using the ratio information and the two blood pressure values (the first blood pressure value and the second blood pressure value).

In Embodiments 2 and 6, the blood pressure estimating section 14 calculates the ratio information based on the reference blood pressure values (the reference diastolic blood pressure, the reference mean blood pressure, and the reference systolic blood pressure) which are previously acquired by a correct adequate technique (for example, the oscillometric method) . The blood pressure estimating section 14 performs the process of estimating the blood pressure while using, for example, (reference mean blood pressure - reference diastolic blood pressure : reference systolic blood pressure - reference mean blood pressure) as the ratio information.

In Embodiments 3 and 7, the blood pressure estimating section 14 calculates the ratio information from the shape of the pulse wave similarly with Embodiments 1 and 5. The blood pressure estimating section 14 corrects the ratio information by using the correction value which is calculated from the reference blood pressure values (the reference diastolic blood pressure, the reference mean blood pressure, and the reference systolic blood pressure), and thereafter estimates the blood pressure.

In Embodiments 4 and 8, the MAP calculator 18 calculates the %MAP in the case where the cuff pressure is 0, from the cuff pressure and transition of the %MAP. The %MAP is a value which is obtained by dividing the average of the area of the waveform, and expressing the quotient in percentage, and may be regarded as a kind of the ratio information. The blood pressure estimating section 14 estimates the third blood pressure value by using the %MAP and the first and second blood pressure values .

In the embodiments, as described above, the measuring apparatus 1 calculates the third blood pressure value by using the first and second blood pressure values which are acquired by using a related-art technique, and the ratio information indicative of the relationships of the difference levels of the blood pressure values. As a blood pressure value of a human being, there are three blood pressure values (the systolic blood pressure, the mean blood pressure, and the diastolic blood pressure). The measuring apparatus 1 calculates the third blood pressure value by using two (first and second blood pressure values) of the three blood pressure values, and the ratio information. Since all the three blood pressure values are not calculated, the measurement time period can be shortened, and the burden imposed on the subject can be reduced (Embodiments 1 to 4). In the other case, the measuring apparatus 1 estimates a blood pressure value which seems to be incorrectly acquired by a related-art measurement method, by using the ratio information (Embodiments 5 to 8). Therefore, it is possible to solve the various problems which are caused in a related-art oscillometric method.

Although, in Embodiments 1 to 8 described above, the ratio information is calculated during measurement, the invention is not limited to this. Usually, it is said that (diastolic blood pressure + pulse pressure $\times$ (1/3)) is equal to the mean blood pressure. Therefore, a specified value, i.e., (mean blood pressure - diastolic blood pressure) : (systolic blood pressure - mean blood pressure) = 1 : 2 may be used as the ratio information. Alternatively, the user of the measuring apparatus 1 may arbitrarily set the specified value. As shown in Non-patent Literature 4, various methods may be considered as the method of calculating the specified value. When the user sets a most adequate specified value in consideration of the condition of the subject and the like, therefore, a desired blood pressure value related to the subject can be appropriately measured.

Although the invention conducted by the inventor has been specifically described based on the embodiments, the invention is not limited to the above-described embodiments, but instead is defined by the appended claims.

For example, a configuration where the systolic blood pressure is estimated in Embodiments 1 to 4, and calculated by the oscillometric method may be employed. That is, the pressure controller 13 does not start the depressurization even after the mean blood pressure is successfully calculated, and continues the pressurization until the systolic blood pressure can be calculated by the oscillometric method. The blood pressure estimating section 14 estimates the systolic blood pressure based on the diastolic blood pressure and the mean blood pressure by the above-described method. The displaying section 15 displays the diastolic and mean blood pressures and the estimated systolic blood pressure. After the systolic blood pressure is successfully calculated by the oscillometric method, the displaying section 15 switches the display so that the estimated systolic blood pressure is overwritten by the systolic blood pressure which is calculated by the oscillometric method.

According to the configuration, the doctor or the like who refers the measuring apparatus 1 can quickly know the general situation of blood pressure values including the systolic blood pressure, and refer the correct systolic blood pressure which is calculated by the oscillometric method.

Apart or all of the above-described processes in the pulse wave measuring section 11, the blood pressure calculator 12, the blood pressure estimating section 14, and the MAP calculator 18 may be realized as computer programs which operate in the measuring apparatus 1. The programs may be stored in a non-transitory computer readable medium of any one of various types, and then supplied to the computer. The non-transitory computer readable medium includes tangible storage media of various types. Examples of the non-transitory computer readable medium are a magnetic recording medium (for example, a flexible disk, a magnetic tape, and a hard disk drive), a magneto-optical recording medium (for example, a magneto-optical disk), a CD-ROM (Read Only Memory), a CD-R, a CD-R/W, a semiconductor memory (for example, a mask ROM, a PROM (Programmable ROM), an EPROM (Erasable PROM), a flash ROM, and a RAM (Random Access Memory)). Alternatively, the programs may be supplied to the computer by means of a transitory computer readable medium of any one of various types. Examples of the transitory computer readable medium are an electrical signal, an optical signal, and an electromagnetic wave. The transitory computer readable medium can supply the programs to the computer through a wired communication path such as a metal wire or an optical fiber, or a wireless communication path.

**Claims**

1. A measuring apparatus (1) comprising:

   a blood pressure calculator (12) which is configured to measure first and second blood pressure values of a subject; and
   a blood pressure estimating section (14) which is configured to estimate a third blood pressure value based on a ratio relating the first, second, and third blood pressure values;
   a pulse wave measuring section (11) which is configured to measure a pulse wave of the subject; and
   a pressure controller (13) which is configured to pressurize a predetermined portion of the subject during measurement of the pulse wave,
   wherein the blood pressure estimating section (14) is configured to calculate the ratio based on a shape of the pulse wave which is measured by the pulse wave measuring section (11),
   wherein the ratio is between a difference between a mean arterial blood pressure and a diastolic blood pressure, and a difference between a systolic blood pressure and the mean arterial blood pressure; and
   the pressure controller (13) is configured to pressurize the predetermined portion of the subject by a pressure which is equal to or higher than a predetermined pressure that is equal to or higher than a venous pressure,
   wherein further the first blood pressure value is a diastolic blood pressure; and the second blood pressure value is a mean blood pressure, and the third blood pressure value is a systolic blood pressure, or the second blood pressure value is a systolic blood pressure, and the third blood pressure value is a mean blood pressure; **characterized in that** the measuring apparatus (1) further comprises:
a MAP calculator (18) which is configured to calculate a change of a %MAP, Percentage of Mean Artery Pressure, from a pressurizing force of the pressure controller (13), and a waveform of the pulse wave, to calculate an estimated value of the %MAP in a case where the pressurization by the pressure controller (13) is cancelled, based on the change of the %MAP and to set the estimated value of the %MAP as the ratio.

2. The measuring apparatus (1) according to claim 1, wherein, when the first and second blood pressure values are to be calculated, the pressure controller (13) is configured to gradually pressurize the predetermined portion of the subject, and, after the calculation is ended, is configured to depressurize.

3. The measuring apparatus (1) according to any one of claims 1 or 2, wherein, during a period of pressurizing the predetermined portion of the subject at a pressure which is equal to or lower than a predetermined pressure that is equal to or lower than a diastolic blood pressure, the pulse wave measuring section (11) is configured to measure the pulse wave.

4. The measuring apparatus (1) according to claim 1, wherein

the blood pressure calculator (12) is configured to previously acquire a reference diastolic blood pressure, a reference mean blood pressure, and a reference systolic blood pressure each of which is a reference value, and the blood pressure estimating section (14) is configured to correct the calculated ratio by using the reference diastolic blood pressure, the reference mean blood pressure, and the reference systolic blood pressure, and thereafter to calculate the third blood pressure value.

5. A measuring method for measuring a blood pressure of a subject, the method comprising the steps of:

calculating first and second blood pressure values of the subject;
estimating a third blood pressure value based on a ratio relating the first, second, and third blood pressure values;
measuring a pulse wave of the subject; and
pressurizing a predetermined portion of the subject during measurement of the pulse wave,
wherein the step of estimating comprises calculating the ratio based on a shape of the pulse wave which is measured at the step of measuring,
wherein the ratio is between a difference between a mean arterial blood pressure and a diastolic blood pressure, and a difference between a systolic blood pressure and the mean arterial blood pressure; and
the step of pressurizing comprises pressurizing the predetermined portion of the subject by a pressure which is equal to or higher than a predetermined pressure that is equal to or higher than a venous pressure,
wherein further the first blood pressure value is a diastolic blood pressure; and the second blood pressure value is a mean blood pressure, and the third blood pressure value is a systolic blood pressure, or the second blood pressure value is a systolic blood pressure, and the third blood pressure value is a mean blood pressure;
**characterized in that** the measuring method further comprises:

calculating a change of a %MAP, Percentage of Mean Artery Pressure, from a pressurizing force, and a waveform of the pulse wave,
calculating an estimated value of the %MAP in a case where the pressurization is cancelled, based on the change of the %MAP and setting the estimated value of the %MAP as the ratio.

6. A program which causes a measuring apparatus (1) according to any one of claims 1 to 4 to execute the measuring method according to claim 5.

7. A non-transitory computer-readable recording medium storing the program according to claim 6.

**Patentansprüche**

1. Messvorrichtung (1), umfassend:

einen Blutdruckkalkulator (12), der konfiguriert ist zum Messen eines ersten und eines zweiten Blutdruckwerts eines Subjekts; und

einen Blutdruckschätzbereich (14), der konfiguriert ist zum Schätzen eines dritten Blutdruckwerts basierend auf einem Verhältnis in Bezug auf den ersten, zweiten und dritten Blutdruckwert;

einen Pulswellenmessbereich (11), der konfiguriert ist zum Messen einer Pulswelle des Subjekts; und

eine Drucksteuereinheit (13), die konfiguriert ist zum Druckbeaufschlagen eines vorbestimmten Abschnitts des Subjekts während Messung der Pulswelle,

wobei der Blutdruckschätzbereich (14) konfiguriert ist zum Berechnen des Verhältnisses basierend auf einer Form der Pulswelle, die von dem Pulswellenmessbereich (11) gemessen wurde,

wobei das Verhältnis zwischen einer Differenz zwischen einem durchschnittlichen arteriellen Blutdruck und einem diastolischen Blutdruck, und einer Differenz zwischen einem systolischen Blutdruck und dem durchschnittlichen arteriellen Blutdruck ist; und

die Drucksteuereinheit (13) konfiguriert ist zum Druckbeaufschlagen des vorbestimmten Abschnitts des Subjekts mit einem Druck, der gleich oder höher ist als ein vorbestimmter Druck, der gleich oder höher ist als ein venöser Druck,

wobei der erste Blutdruckwert weiter ein diastolischer Blutdruck ist; und der zweite Blutdruckwert ein durchschnittlicher Blutdruck ist und der dritte Blutdruckwert ein systolischer Blutdruck ist, oder der zweite Blutdruckwert ein systolischer Blutdruck ist und der dritte Blutdruckwert ein durchschnittlicher Blutdruck ist;

**dadurch gekennzeichnet ist, dass**

die Messvorrichtung (1) weiter Folgendes umfasst:

einen MAP-Kalkulator (18), der konfiguriert ist zum Berechnen einer Änderung eines %MAP, Prozentsatz des durchschnittlichen Arteriendrucks, aus einer Druckbeaufschlagungskraft der Drucksteuereinheit (13), und einer Wellenform der Pulswelle zum Berechnen eines Schätzwerts des %MAP, wenn die Druckbeaufschlagung durch die Drucksteuereinheit (13) aufgehoben wird, basierend auf der Änderung des %MAP, und zum Einstellen des Schätzwerts des %MAP als das Verhältnis.

2. Messvorrichtung (1) nach Anspruch 1, wobei, wenn der erste und der zweite Blutdruckwert berechnet werden sollen, die Drucksteuereinheit (13) konfiguriert ist zum schrittweisen Druckbeaufschlagen des vorbestimmten Abschnitts des Subjekts, und nachdem die Berechnung beendet ist, konfiguriert ist zum Druckentspannen.

3. Messvorrichtung (1) nach einem der Ansprüche 1 oder 2, wobei während einer Periode der Druckbeaufschlagung des vorbestimmten Abschnitts des Subjekts mit einem Druck, der gleich oder niedriger als ein vorbestimmter Druck ist, der gleich oder niedriger ist als ein diastolischer Blutdruck, der Pulswellenmessbereich (11) konfiguriert ist zum Messen der Pulswelle.

4. Messvorrichtung (1) nach Anspruch 1, wobei

der Blutdruckkalkulator (12) konfiguriert ist zum vorausgehenden Erfassen eines diastolischen Referenzblutdrucks, eines durchschnittlichen Referenzblutdrucks und eines systolischen Referenzblutdrucks, die jeweils ein Referenzwert sind, und

der Blutdruckschätzbereich (14) konfiguriert ist zum Korrigieren des errechneten Verhältnisses durch Verwenden des diastolischen Referenzblutdrucks, des durchschnittlichen Referenzblutdrucks und des systolischen Referenzblutdrucks, und danach zum Berechnen des dritten Blutdruckwerts.

5. Messverfahren zum Messen eines Blutdrucks eines Subjekts, wobei das Verfahren die folgenden Schritte umfasst:

Berechnen eines ersten und eines zweiten Blutdruckwerts des Subjekts;

Schätzen eines dritten Blutdruckwerts basierend auf einem Verhältnis in Bezug auf den ersten, zweiten und dritten Blutdruckwert;

Messen einer Pulswelle des Subjekts; und

Druckbeaufschlagung eines vorbestimmten Abschnitts des Subjekts während Messung der Pulswelle,

wobei der Schritt des Schätzens Berechnen des Verhältnisses basierend auf einer Form der Pulswelle umfasst, die in dem Schritt des Messens gemessen wurde,

wobei das Verhältnis zwischen einer Differenz zwischen einem durchschnittlichen arteriellen Blutdruck und einem diastolischen Blutdruck, und einer Differenz zwischen einem systolischen Blutdruck und dem durchschnittlichen arteriellen Blutdruck ist; und

der Schritt des Druckbeaufschlagens Druckbeaufschlagen des vorbestimmten Abschnitts des Subjekts mit einem Druck umfasst, der gleich oder höher ist als ein vorbestimmter Druck, der gleich oder höher ist als ein venöser Druck,

wobei der erste Blutdruckwert weiter ein diastolischer Blutdruck ist; und der zweite Blutdruckwert ein durchschnittlicher Blutdruck ist und der dritte Blutdruckwert ein systolischer Blutdruck ist, oder der zweite Blutdruckwert ein systolischer Blutdruck ist und der dritte Blutdruckwert ein durchschnittlicher Blutdruck ist;

**dadurch gekennzeichnet ist, dass**

das Messverfahren weiter Folgendes umfasst:

Berechnen einer Änderung eines %MAP, Prozentsatz des durchschnittlichen Arteriendrucks, aus einer Druckbeaufschlagungskraft und einer Wellenform der Pulswelle, Berechnen eines Schätzwerts des %MAP, wenn die Druckbeaufschlagung aufgehoben wird, basierend auf der Änderung des %MAP, und Einstellen des Schätzwerts des %MAP als das Verhältnis.

6. Programm, das bewirkt, dass eine Messvorrichtung (1) nach einem der Ansprüche 1 bis 4 das Messverfahren nach Anspruch 5 ausführt.

7. Nichttransitorisches computerlesbares Speichermedium, das das Programm nach Anspruch 6 speichert.

**Revendications**

1. Appareil de mesure (1) comprenant :

un calculateur (12) de pression artérielle qui est configuré pour mesurer des première et deuxième valeurs de pression artérielle d'un sujet ; et
une section d'estimation (14) de pression artérielle qui est configurée pour estimer une troisième valeur de pression artérielle sur la base d'un rapport se rapportant aux première, deuxième et troisièmes valeurs de pression artérielle ;
une section de mesure (11) d'onde de pouls qui est configurée pour mesurer une onde de pouls du sujet ; et
un dispositif de commande (13) de pression qui est configuré pour pressuriser une partie prédéterminée du sujet pendant la mesure de l'onde de pouls,
dans lequel la section d'estimation (14) de pression artérielle est configurée pour calculer le rapport sur la base d'une forme de l'onde de pouls qui est mesurée par la section de mesure (11) d'onde de pouls,
dans lequel le rapport est entre une différence entre une pression artérielle moyenne et une pression artérielle diastolique, et une différence entre une pression artérielle systolique et la pression artérielle moyenne ; et
le dispositif de commande (13) de pression est configuré pour pressuriser la partie prédéterminée du sujet par une pression qui est égale ou supérieure à une pression prédéterminée qui est égale ou supérieure à une pression veineuse,
dans lequel en outre la première valeur de pression artérielle est une pression artérielle diastolique ; et la deuxième valeur de pression artérielle est une pression artérielle moyenne, et la troisième valeur de pression artérielle est une pression artérielle systolique, ou la deuxième valeur de pression artérielle est une pression artérielle systolique, et la troisième valeur de pression artérielle est une pression artérielle moyenne ; **caractérisé en ce que**
l'appareil de mesure (1) comprend en outre :
un calculateur (18) de MAP qui est configuré pour calculer un changement d'un %MAP, pourcentage de pression artérielle moyenne, à partir d'une force de pressurisation du dispositif de commande (13) de pression, et d'une forme d'onde de l'onde de pouls, pour calculer une valeur estimée du %MAP dans un cas où la pressurisation par le dispositif de commande (13) de pression est annulée, sur la base du changement du %MAP et pour définir la valeur estimée du %MAP comme rapport.

2. Appareil de mesure (1) selon la revendication 1, dans lequel, lorsque les première et deuxième valeurs de pression artérielle doivent être calculées, le dispositif de commande (13) de pression est configuré pour pressuriser progressivement la partie prédéterminée du sujet, et, après la fin du calcul, est configuré pour dépressuriser.

3. Appareil de mesure (1) selon l'une quelconque des revendications 1 ou 2, dans lequel, pendant une période de pressurisation de la partie prédéterminée du sujet à une pression qui est égale ou inférieure à une pression prédéterminée qui est égale ou inférieure à une pression artérielle diastolique, la section de mesure (11) d'onde de pouls est configurée pour mesurer l'onde de pouls.

4. Appareil de mesure (1) selon la revendication 1, dans lequel

le calculateur (12) de pression artérielle est configuré pour acquérir au préalable une pression artérielle diastolique de référence, une pression artérielle moyenne de référence et une pression artérielle systolique de référence, chacune d'elles étant une valeur de référence, et

la section d'estimation (14) de pression artérielle est configurée pour corriger le rapport calculé en utilisant la pression artérielle diastolique de référence, la pression artérielle moyenne de référence et la pression artérielle systolique de référence, et par la suite pour calculer la troisième valeur de pression artérielle.

**5.** Procédé de mesure pour mesurer une pression artérielle d'un sujet, le procédé comprenant les étapes consistant à :

calculer des première et deuxième valeurs de pression artérielle du sujet ;

estimer une troisième valeur de pression artérielle sur la base d'un rapport se rapportant aux première, deuxième et troisièmes valeurs de pression artérielle ;

mesurer une onde de pouls du sujet ; et

pressuriser une partie prédéterminée du sujet pendant la mesure de l'onde de pouls,

dans lequel l'étape d'estimation comprend un calcul du rapport sur la base d'une forme de l'onde de pouls qui est mesurée lors de l'étape de mesure,

dans lequel le rapport est entre une différence entre une pression artérielle moyenne et une pression artérielle diastolique, et une différence entre une pression artérielle systolique et la pression artérielle moyenne ; et

l'étape de pressurisation comprend une pressurisation de la partie prédéterminée du sujet par une pression qui est égale ou supérieure à une pression prédéterminée qui est égale ou supérieure à une pression veineuse,

dans lequel en outre la première valeur de pression artérielle est une pression artérielle diastolique ; et la deuxième valeur de pression artérielle est une pression artérielle moyenne, et la troisième valeur de pression artérielle est une pression artérielle systolique, ou la deuxième valeur de pression artérielle est une pression artérielle systolique, et la troisième valeur de pression artérielle est une pression artérielle moyenne ; **caractérisé en ce que**

le procédé de mesure comprend en outre :

un calcul d'un changement d'un %MAP, pourcentage de pression artérielle moyenne, à partir d'une force de pressurisation, et d'une forme d'onde de l'onde de pouls, un calcul d'une valeur estimée du %MAP dans un cas où la pressurisation est annulée, sur la base du changement du %MAP et une définition de la valeur estimée du %MAP comme rapport.

**6.** Programme qui amène un appareil de mesure (1) selon l'une quelconque des revendications 1 à 4 à exécuter le procédé de mesure selon la revendication 5.

**7.** Support d'enregistrement non transitoire lisible par ordinateur stockant le programme selon la revendication 6.

*FIG. 1*

## FIG. 2

EP 3 069 655 B1

# FIG. 3

VESSEL INTERNAL PRESSURE
(BLOOD PRESSURE - CUFF PRESSURE)

DEPRESSURIZATION

E

D

C

B

A

0

ARTERIAL BLOOD
VESSEL VOLUME

E

D

C

B

A

CUFF
PRESSURE

CUFF
PRESSURE

VOLUMETRIC
CHANGE

A    B    C    D    E

FIG. 4

## FIG. 5

EP 3 069 655 B1

# FIG. 6(A)

# FIG. 6(B)

FIG. 7

EP 3 069 655 B1

## FIG. 8

FIG. 9

AVERAGE OF AREA

AMPLITUDE

%MAP

NORMAL WAVEFORM

100%

38%

0%

NARROWED WAVEFORM

100%

50%

0%

## FIG. 10

EP 3 069 655 B1

# FIG. 11

AMPLITUDE OF
PULSE WAVE

UPPER RIGHT ARM

CUFF PRESSURE

AMPLITUDE OF
PULSE WAVE

UPPER LEFT ARM

CUFF PRESSURE

AMPLITUDE OF
PULSE WAVE

RIGHT ANKLE

CUFF PRESSURE

AMPLITUDE OF
PULSE WAVE

LEFT ANKLE

CUFF PRESSURE

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2014230671 A
- JP 10276994 A
- US 2005119578 A1
- EP 1302155 A2
- US 2010298726 A1

### Non-patent literature cited in the description

- **SHIMAZU HIDEAKI.** *Kenko o Sasaeru Iryokiki Kantan de Oku no Fukai Ketsuatsu Sokutei, Clin Eng,* vol. 9 (1), 26-33
- **YOSHIKAWA KIMIHIKO.** *Heisokusei Domyaku Kokasho Shindan ni Okeru ABI Sokutei to Myakuhakei no Juyosei, DAI 8 KAI RINSHO KETSUATSU MYAKUHA KENKYUKAI SHOREI KENTOKAI,* vol. 3, 58, , 60
- *Colin Information Service,* 23 February 2015, http://www.colin.omron.co.jp/colininfo/pdf/11.pdf
- **DENIS CHEMLA et al.** aortic pressure is the geometric mean of systolic and diastolic aortic pressure in resting humans. *aortAppl Phusiol,* 2005, vol. 99, 2278-2284